# EUROPEAN PATENT APPLICATION

(11) **EP 3 960 210 A1**
(43) Date of publication of application: **02.03.2022**
(21) Application number: 21191321.5
(22) Date of filing: 13.08.2021
(51) Int. Cl.: A61L 2/10, A61L 2/26, B65D 57/00, B65D 33/02, B65D 85/18

(54) **CLEANING OF FACE MASKS**

(30) Priority: 25.08.2020 BE 202005589
(71) Applicant: Elektrotechniek Verno B.V., 9140 Temse (BE)
(72) Inventor: NOENS, Philippe, 9140 TEMSE (BE)
(74) Representative: Arnold & Siedsma

(57) **Abstract**

Device for cleaning face masks, the device comprising a housing with an inlet opening and an outlet opening, wherein a throughfeed mechanism is provided for carrying the face masks from the inlet opening to the outlet opening along a throughfeed path, wherein cleaning means are provided in the housing all around the throughfeed path in a manner such that the cleaning has an effect from substantially all perpendicular directions of incidence to the throughfeed path, wherein the device further comprises an inlet screening and outlet screening comprising a supply opening and a discharge opening, wherein the throughfeed mechanism is further provided to carry the face masks from the supply opening to the inlet opening and is provided to carry the face masks from the outlet opening to the discharge opening, wherein the supply opening and the discharge opening lie at a distance from the throughfeed path in order to screen a surrounding area from the cleaning means.

## Description

The invention relates to the cleaning of face masks. For this purpose a device is provided and a method is provided.

The invention came into being during a crisis in which there was a shortage of high-quality face masks. During this time it quickly became apparent that a face mask can only be used safely for a short period of time, after which the face mask must be replaced. The primary purpose of a face mask is to protect the wearer and/or the environment against transmission of microorganisms and viruses. For this purpose the face mask forms a barrier to microorganisms and viruses. This primary purpose of the face mask is only achieved when the face mask is free of biological contamination. Biological contamination builds up due to use of the face mask, after which the face mask must be replaced.

High-quality face masks are not intended to be disposed of after a single use, but to be used repeatedly. For this purpose a good cleaning is necessary. The cleaning has the primary object of removing the biological contamination from the face mask. Biological contamination is difficult to detect, making it difficult to check whether a face mask is free of contamination. Incomplete or insufficient cleaning can therefore create a false sense of security, and should be avoided.

It must be possible to guarantee the quality of the cleaning, which is directly related to the degree to which a face mask has been cleaned fully and sufficiently, over the whole surface of the face mask. In the event that a small part of the surface of the face mask was cleaned to a lesser standard, biological contaminations may still be present on the face mask and contaminate its wearer or a bystander. Inferior cleaning of only a small part of the surface therefore has negative consequences for the whole face mask.

It is an object of the invention to provide a device and method wherein the quality of cleaning of the face mask can be improved.

For this purpose the invention provides a device for cleaning face masks, the device comprising a housing with an inlet opening and an outlet opening, wherein a throughfeed mechanism is provided for carrying the face masks from the inlet opening to the outlet opening along a throughfeed path, wherein cleaning means are provided in the housing all around the throughfeed path in a manner such that the cleaning has an effect from substantially all perpendicular directions of incidence to the throughfeed path, wherein the device further comprises an inlet screening and outlet screening comprising a supply opening and a discharge opening, wherein the throughfeed mechanism is further provided to carry the face masks from the supply opening to the inlet opening and is provided to carry the face masks from the outlet opening to the discharge opening, wherein the supply opening and the discharge opening lie at a distance from a central axis of the throughfeed path in order to screen a surrounding area from the cleaning means.

By providing a throughfeed path in a housing and providing cleaning means in the housing all around the path, such that cleaning has an effect from substantially all perpendicular directions of incidence to the throughfeed path, arbitrariness is avoided in the cleaning. More specifically, it can hereby be guaranteed that the cleaning means have acted on the face mask from at least all perpendicular angles of incidence. By moving the face masks along the throughfeed path while the cleaning means act on the throughfeed path perpendicularly from all directions it is further automatically also achieved the cleaning means act on the face masks at oblique angles of incidence. The specific structure of the device also avoids arbitrariness for the action at oblique angles of incidence. In other words, with this structure a substantially wholly omnidirectional action of the cleaning means on the face masks is achieved. It is particularly because face masks have a complex three-dimensional form that the omnidirectional action ensures that the whole surface of the face mask can be reached directly by the cleaning means. In other words, an undesired shadow zone cannot result.

In order to enable a substantially omnidirectional action of the cleaning means inlet screening and outlet screening are provided. This inlet screening and outlet screening ensure that the cleaning means no longer have any effect outside the housing. Because the cleaning means are primarily configured to clean or remove biological contaminations such as microorganisms and viruses, the cleaning means are harmful to humans and animals. Throughfeed means are provided which carry the face masks from a supply opening, through the housing, to a discharge opening. The supply opening and the discharge opening here lie at a distance from the central axis of the throughfeed path. In other words, the object is for the cleaning means to be able to reach neither the supply opening nor the discharge opening. The cleaning means have no direct action at the position of the supply opening and the discharge opening owing to the specific structure of the device.

The combination of features of the device allows an improvement of the quality of the cleaning.

The face masks preferably each have on either side a fastening element, and the throughfeed mechanism has for each face mask at least two, preferably at least three protrusions at a mutual distance, such that the face mask can be connected via its fastening elements to the protrusions so as to be suspended between the protrusions. By connecting the face mask to two, preferably three protrusions, the face mask can be suspended with its base body in floating manner. Suspending the face mask in floating manner prevents elements of the device from inhibiting direct action of the cleaning means on the face mask. If the face mask were for instance placed on a conveyor belt, the contact zones between the conveyor belt and the face mask could not be cleaned directly. This problem is solved by connecting the face mask with fastening elements between two or three protrusions located at a mutual distance.

The throughfeed mechanism preferably comprises a plurality of frames with the protrusions, wherein each frame is provided to hold a predetermined number of face masks, wherein the throughfeed mechanism further comprises a transport means to which the frames can be connected in order to move the frames with the face masks along the throughfeed path. The frame facilitates the logistical process before and after throughfeed of the face masks through the device. Each face mask can be suspended on a frame, automatically or manually, after which the frames with the face masks can be stored and transported further before carrying the frames with the face masks through the device. Transport and storage of the frames with face masks can also be at least partially automated after throughfeed. This allows efficient use of the device.

The frame is preferably formed as a framework, wherein the protrusions are provided on the framework so that the face mask can be suspended within boundaries of the framework. The face mask can then be suspended in the framework and is freely accessible from all directions. This allows the cleaning means to act omnidirectionally on the face mask in simple manner.

The frame is preferably magnetically compatible with the transport means in order to connect the frame magnetically to the transport means. A magnetic coupling and uncoupling of frame and transport means can be realized and automated in simple manner.

The throughfeed mechanism preferably comprises two parallel throughfeed strings between which the face masks can be secured. The transport means preferably comprises the throughfeed strings. The throughfeed strings can be embodied as solid or woven metal wire, optionally with a plastic coating or casing.

The throughfeed mechanism preferably comprises at the position of each of the inlet opening and the outlet opening at least one bend element for changing a throughfeed direction. Providing a bend realizes that the cleaning means can reach neither the supply opening nor the discharge opening.

The cleaning means preferably comprises at least one UV-C lamp. A UV-C lamp is known as biological cleaning means. Depending on the dose and operating intensity a UV-C lamp will kill almost all microorganisms and viruses so that a biological cleaning is achieved.

The at least one UV-C lamp is preferably mounted movably in the housing such that the UV-C lamp is movable all around the central axis so as to change an angle of incidence of radiation of the UV-C lamp onto the throughfeed path. The at least one UV-C lamp preferably comprises a whole number N of UV-C lamps, wherein N is greater than or equal to 2 and wherein the UV-C lamps are positioned around the central axis with an angular intermediate distance of 360 degrees divided by N. A UV-C lamp can be seen as a point source. When such a point source is moved through 360 degrees around an axis over a period of time, it is achieved that light from the point source is incident on the axis from all perpendicular directions within the period of time. The skilled person will appreciate that a UV-C lamp can alternatively be provided in annular manner, optionally in a plurality of segments, around the central axis in order to provide incidence of light from all perpendicular directions.

The N UV-C lamps are preferably mounted on a structure which is bearing-mounted rotatably in the housing, which structure is provided for reciprocal rotation about the central axis between two end positions lying at a mutual angular intermediate distance of at least 360 degrees divided by N. By providing for instance four UV-C lamps and having them rotate reciprocally through 90 degrees of the structure on which the four lamps are provided, it is realized in simple manner that light from the UV-C lamps is incident on the central axis from all perpendicular directions.

The housing preferably further comprises a sensor for measuring an operating intensity of the cleaning means. The sensor is preferably operatively coupled to the throughfeed mechanism so as to at least partially control a throughfeed speed on the basis of an output of the sensor. Because the cleaning means are in a housing, it cannot be checked visually whether the cleaning means are operating correctly. By providing a sensor and directly measuring the action of the cleaning means it can be guaranteed that the face masks have also been subjected to a predetermined dose of the cleaning means. Depending on the operating intensity of the cleaning means, the throughfeed speed can here be adjusted in order to guarantee that each face mask has received a minimal cleaning. This makes it possible to offer a quality guarantee. The sensor also allows immediate detection of incorrect operation or malfunction of a cleaning means.

A loading zone and an unloading zone are preferably provided adjacently of respectively the supply opening and the discharge opening. Via the loading and unloading zone the device can be respectively loaded and unloaded manually or automatically in simple manner.

The invention further relates to a method for cleaning face masks, the method comprising of:
- feeding the face masks through a housing using a throughfeed mechanism from an inlet opening to an outlet opening along a throughfeed path;
- cleaning in the housing via cleaning means which are provided all around the throughfeed path in a manner such that the cleaning has an effect from substantially all perpendicular directions of incidence to the throughfeed path;
- screening off the cleaning means via an inlet screening and outlet screening comprising a supply opening and a discharge opening, wherein the throughfeed further comprises of carrying the face masks from the supply opening to the inlet opening and from the outlet opening to the discharge opening, wherein the supply opening and the discharge opening lie at a distance from a central axis of the throughfeed path.

The advantages and effects of the method are analogous to the above described advantages and effects of the device.

The method preferably further comprises of measuring an operating intensity of the cleaning means during throughfeed. The cleaning means preferably comprises a whole number N of UV-C lamps, wherein N is greater than or equal to 2 and wherein the UV-C lamps are positioned around the central axis with an angular intermediate distance of 360 degrees divided by N, and wherein the step of cleaning comprises of reciprocally rotating the N UV-C lamps about the central axis through an angular distance of at least 360 degrees divided by N. The advantages and effects of these preferences of the method are analogous to the above described advantages and effects of the analogous preferences of the device.

The invention will now be further described on the basis of exemplary embodiments shown in the drawings.

In the drawing:
figure 1 shows a schematic longitudinal section of a device according to an embodiment of the invention;
figure 2 shows a schematic cross-section of a housing from a device according to an embodiment of the invention;
figures 3A-3D show aspects of a throughfeed mechanism from a device according to an embodiment of the invention; and
figure 4 shows a perspective view of a device according to an embodiment of the invention.

The same or similar elements are designated in the drawing with the same reference numerals.

The invention came into being in the search for a solution for cleaning face masks. The invention has for its object here to provide a device and method whereby a quality guarantee can be offered in respect of the cleaning. This means that all aspects of the cleaning are subject to as little arbitrariness as possible, and are preferably also measurable. An important aspect of the cleaning relates to the supply, discharge and the manipulation during cleaning. When a face mask must for instance be turned over in order to be cleaned on both sides, this turning over will typically create an uncertainty in respect of the cleaning. This is because such turning over will in each case enable direct or indirect contact between an already cleaned side and an as yet uncleaned side of the face mask. It is hereby never possible to guarantee that the cleaning is complete and uncompromised.

The principles that are applied in the device and method according to the invention have been found particularly advantageous for the cleaning of face masks. The skilled person will however appreciate that, additionally, other objects can also be cleaned by means of this device and method. This description therefore provides an explicit basis for the cleaning of objects. Just as a face mask, the objects preferably have a functional zone and one or more fastening elements. The fastening elements are preferably provided in order to place and/or hold the functional zone in a predetermined position during use of the object. In the case of a face mask the fastening elements are typically formed by two loops which are attached on either side of the face mask and are provided to engage behind the ears of a user in order to hold the face mask against the face of the user. Other objects which can be cleaned using the device according to the invention are glasses such as safety goggles, and hearing protection. By scaling and adjusting the form and setup of the device the range of possible applications becomes wider still, so that almost all utility articles which are resistant to UV radiation and which benefit from being disinfected qualify as object.

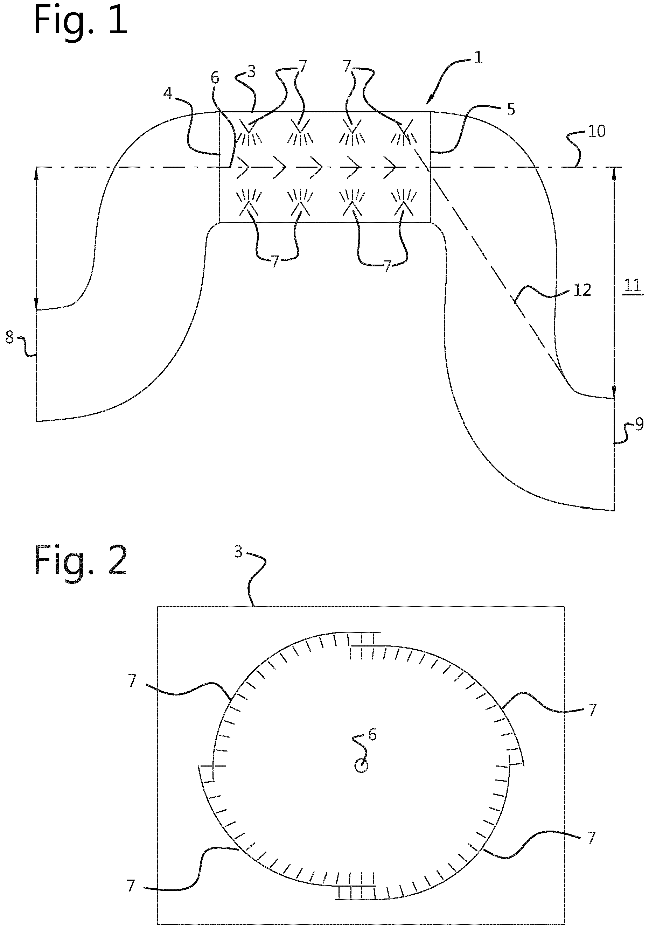

Figure 1 shows a longitudinal section of a structure of a device 1 according to an embodiment of the invention. The device has a housing 3. The housing 3 is preferably provided to screen objects in housing 3 from the surrounding area. As will be discussed below in more detail, cleaning means 7 are provided in housing 3. Housing 3 is here also provided to physically and functionally screen the cleaning means 7 from the surrounding area. Functional screening is understood to mean that the cleaning means have no noticeable operational effect outside the housing. When the cleaning means operate on the basis of radiation, the housing will block the radiation so that the radiation is unable to reach the surrounding area, or at least cannot reach it directly.

Housing 3 has an inlet opening 4 and an outlet opening 5. A throughfeed path 6 extends between inlet opening 4 and outlet opening 5. Face masks can be carried via inlet opening 4, along throughfeed path 6 to outlet opening 5. Housing 3 is preferably formed substantially symmetrically around the throughfeed path 6. It will be apparent that, at the position of its inlet opening 4 and outlet opening 5, the housing does not screen off the cleaning means. For this purpose device 1 is provided with an inlet screening and outlet screening. The inlet screening and outlet screening can be formed by a lock with gates or valves which are not open simultaneously. A lock allows a discontinuous supply and discharge of face masks. Alternatively, as shown in the figure, a bend is provided at the position of each of the inlet opening 4 and outlet opening 5, which bend is screened off by a covering. This covering can be formed by an extension piece of housing 3.

The inlet screening and outlet screening have respectively a supply opening 8 and discharge opening 9. The face masks to be cleaned can be introduced into the device via the supply opening 8. Cleaned face masks can be removed from the device via the discharge opening 9. Supply opening 8 is connected to the inlet opening 4 of the housing. Outlet opening 5 is connected to the discharge opening 9. While cleaning means typically do still have an at least partial effect at the position of inlet opening 4 and outlet opening 5, the inlet screening and outlet screening are formed such that the cleaning means no longer have any noticeable effect at the position of supply opening 8 and discharge opening 9. In the shown embodiment the bend is formed such that cleaning means 7 are unable to reach the supply and/or discharge opening 8, 9 in a straight line, shown in figure 1 as line 12.

In the shown embodiment supply opening 8 and discharge opening 9 lie at a distance from the central axis 10 which is determined by the throughfeed path 6. This distance is designated in figure 1 with arrow 11. The skilled person will appreciate that a meander bend, wherein supply opening 8 and discharge opening 9 do lie on the central axis 10, but wherein there is a bend between supply opening 8 and inlet opening 4 and between discharge opening 9 and outlet opening 5, is likewise possible. In other words, the inlet screening and outlet screening can take the form of a labyrinth. When lock gates are provided, no bend need be provided between supply opening 8 and inlet opening 4 and between discharge opening 9 and outlet opening 5. The skilled person will understand the implications of the inlet screening and outlet screening, and can apply them in the device on the basis of the operational preferences of the device.

Cleaning means 7 are placed in housing 3 in a manner such that the cleaning means have a direct or indirect effect from substantially all perpendicular directions of incidence to throughfeed path 6 at any moment or for a predetermined period of time. This can be realized in different ways, as will be apparent from the description below. Cleaning means 7 also extend in the direction of throughfeed path 6 over a distance which preferably amounts to at least half, more preferably at least two thirds of the length of throughfeed path 6.

Figure 2 shows a cross-section of the housing 3. In the embodiment shown in figure 2 the cleaning means take the form of UV lamps, preferably UV-C lamps. UV-C lamps and their effect on microorganisms and viruses are known, and are therefore not explained further in this description. Figure 2 shows four tubular UV-C lamps, each extending through at least 90 degrees around throughfeed path 6. Owing to their positioning, the four lamps together ensure that radiation is incident on throughfeed path 6 at every perpendicular angle. The skilled person will appreciate that the lamps can be placed in one plane transversely of the throughfeed path 6, but that the lamps can also be placed along a spiral, i.e. with a component in the longitudinal direction, through the at least 90 degrees. The lamps are preferably tubular and can be straight (not shown) or curved. The skilled person will appreciate that when a straight tubular lamp extends through an angle, the central part of the tubular lamp lies closer to throughfeed path 6 than the outer ends of the tubular lamp. The intensity will hereby not be constant, but radiation will be incident from every angle. In any case, when the lamps are mounted in stationary manner in housing 2, N lamps are provided and each lamp extends through 360/N degrees in order to achieve the effect of the radiation being incident on throughfeed path 6 at at least every perpendicular angle. Because radiation typically does not come from the lamps in just one direction, the radiation will additionally also be incident at a plurality of angles other than the perpendicular angle to throughfeed path 6. This improves the action of the cleaning means. More specifically, the effect is that when cleaning means have an effect at least from all perpendicular directions to throughfeed path 6, shadows (or indirect effect) of the cleaning means is avoided, even when the surface of the face mask has a complex three-dimensional form.

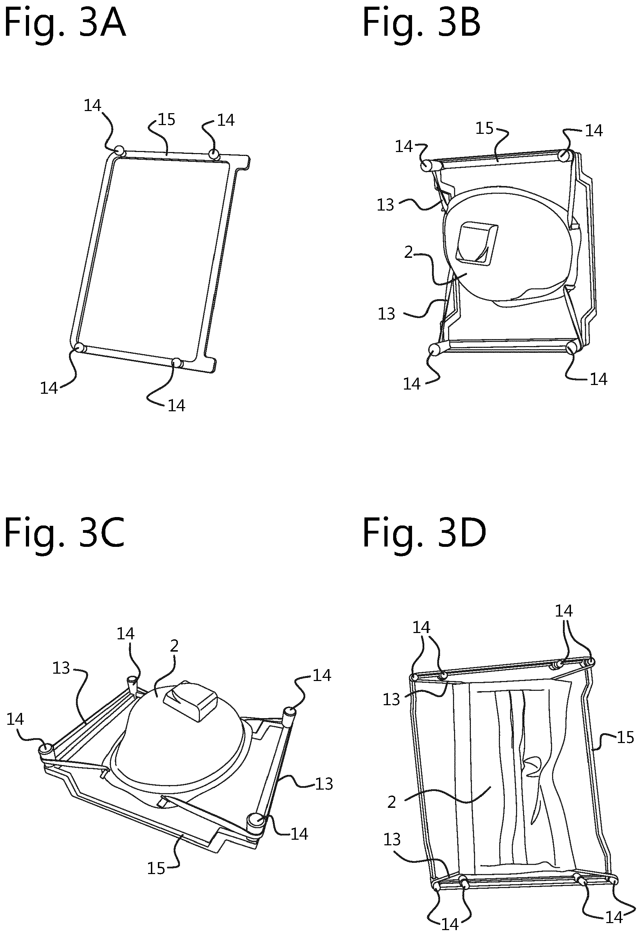

Figure 3A shows a frame 15 for a face mask. The frame forms a framework, preferably rectangular, wherein a protrusion 14 is provided close to each corner. The size of frame 15 and the position of protrusions 14 are selected on the basis of the face mask to be cleaned. The skilled person will appreciate here that an embodiment with four protrusions is just an example. The minimum number of protrusions for securing an object inside a frame at a determined position is three protrusions. Providing more than three protrusions increases a flexibility in respect of the object to be secured and/or the stability of secured objects.

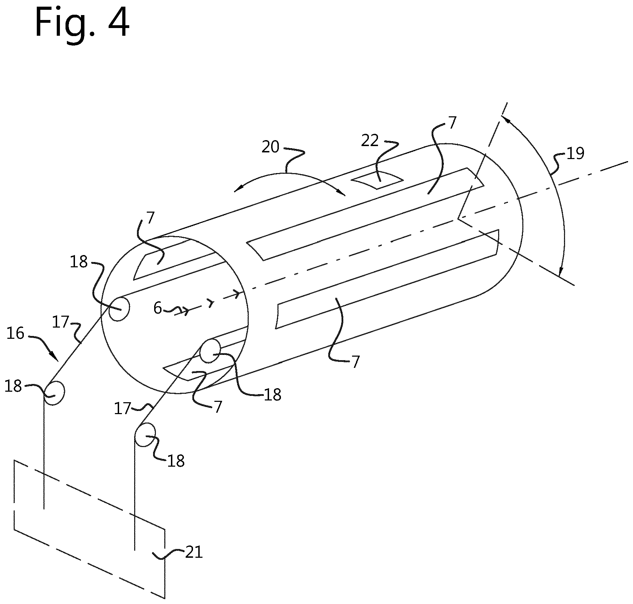

The frame can preferably be coupled to a throughfeed mechanism, which is discussed below in more detail with reference to figure 4. The coupling can be realized on the basis of friction. According to a very simple embodiment, the frames are placed on a movable element of the throughfeed mechanism and the frame remains thereon due to the force of gravity, and is co-displaced through the device. Alternatively, a clamping connection or hook connection is provided. Preferably provided as further alternative is a magnetic connection. The frame is preferably provided here with one or more magnets and the throughfeed mechanism with a ferromagnetic material in order to realize simple coupling and uncoupling of the frame to the throughfeed mechanism.

Figure 3B shows a frame 15 and a face mask 2 which is connected to the frame 15. Frame 15 forms a framework and has a plurality of protrusions 14. Face mask 2 has a functional zone and two fastening elements 13, which are provided on either side of the functional zone. The fastening elements 13 are for instance cords or pieces of elastic whereby the face mask can be held in front of the mouth and nose of a wearer. The cords or pieces of elastic are provided to engage behind the ears or behind the head of the wearer thereof. These same cords or pieces of elastic are placed behind the protrusions 14 and the face mask is thus connected to the frame. Figure 3B shows that a face mask can be suspended inside the frame by hooking fastening elements 13 behind the protrusions. It will be apparent here that the term 'inside the frame' relates to one projection of face mask and frame, more specifically the projection perpendicularly of the frame in the form of a framework. As will be apparent from figures 3B and 3C, the face mask will not be able to fall 'inside' the frame in a lateral projection due to its three-dimensional form.

Figure 3C shows the same frame 15 and face mask 2 as figure 3B, but in perspective view. It is apparent from this figure that, due to its three-dimensional form, the face mask may protrude above the frame when the face mask is connected to the frame. This figure 3C also shows that the frame need not necessarily be formed in one plane, but that different sides of the frame can be formed in different planes. When segments of the frame which fall between two throughfeed strings (further elucidated hereinbelow) lie lower than the segments which come to lie on the throughfeed strings, positioning of the frame on the throughfeed strings is facilitated.

Figure 3D shows an alternative embodiment of a frame 15. A different face mask 2 than the face mask shown in figures 3B and 3C is secured in frame 15. Figure 3D further shows an embodiment wherein the frame has eight protrusions. By providing eight protrusions face masks with different dimensions and specifications can be connected to the frame. Big face masks, size large, can thus be connected to the four outermost protrusions. Small face masks, size small, can also be connected to the four innermost protrusions. Intermediate sizes can be connected to other combinations of protrusions. The skilled person will appreciate that this also allows different types of face mask to be secured to the same frame, wherein the protrusions 14 of the frame which are used to secure the face mask depend on the size and specifications of the face mask. Coupling elements such as magnets can be provided at the position of one or more protrusions. Alternatively, magnets can be provided in openings specially provided for this purpose.

Figure 4 shows a preferred embodiment of a throughfeed mechanism 16 and a setup of cleaning means for application in the device according to the invention. The setup and operation of the cleaning means 7 will be elucidated first hereinbelow. Following this, the setup and operation of throughfeed mechanism 16 will be elucidated. The skilled person will appreciate that the two can be applied independently of each other and that the combination, which is shown, forms a further preferred embodiment.

In figure 4 the cleaning means 7 are formed by straight tubular UV-C lamps. The UV-C lamps are placed parallel to the throughfeed path. In this embodiment four UV-C lamps are provided, each placed 90° apart relative to the throughfeed path, illustrated in the figure with arrow 20. In stationary state this setup does not meet the criterium of the cleaning which has an effect from substantially all perpendicular directions of incidence to the throughfeed path. This is because, in stationary state, the cleaning would only have an effect from four perpendicular directions of incidence which are separated by 90 degrees. The UV-C lamps are however placed in a frame which can rotate reciprocally through at least 90°, illustrated in the figure with arrow 19. The skilled person will appreciate here that alternative setups with more or fewer lamps are possible, wherein the frame must be rotatable through at least 360 degrees divided by the number of lamps. By rotating reciprocally over a period of time T radiation will have had an effect from all perpendicular directions of incidence to the throughfeed path when considered halfway through the period of time T. The speed at which the frame rotates reciprocally is adjusted to the speed with which face masks are carried through the housing, this such that half of the above stated period of time T is at least greater than or equal to the time T1 needed by a face mask to travel a distance equal to the length of the UV-C lamps. Within this ratio it can be guaranteed that the UV-C lamps have acted on the face mask from all directions.

In order to guarantee the quality of the cleaning a sensor 22 is preferably placed inside the housing. The sensor 22 preferably measures the effect of the cleaning means continuously. When the cleaning means are formed by UV-C lamps, sensor 22 preferably measures the radiation intensity continuously. UV-C lamps have a warm-up time. UV-C lamps have further been found to degrade over time, whereby the operating intensity decreases. In order to compensate therefor the operating intensity is continuously monitored via the sensor 22, and a throughfeed speed of face masks is adjusted to the output of sensor 22. A skilled person can hereby set a minimal operational effect of the cleaning means on a face mask. The extent to which the operational effect of the cleaning means has had an effect on a face mask can be monitored by a skilled person via the sensor, which measures the operating intensity, and the throughfeed speed of the face masks, which determines the period of time during which a face mask is exposed to the cleaning means. The skilled person can then either adjust the throughfeed speed or adjust the operating intensity, or both, in order to ensure that face masks receive an optimal cleaning. The capacity of the device can hereby further be optimized, while the quality remains guaranteed.

Figure 4 further shows an embodiment of a throughfeed mechanism 16 which has two throughfeed strings 17. The throughfeed strings 17 run at a mutual distance and substantially parallel, and in the housing also run parallel to the throughfeed path. Throughfeed path 6 preferably runs roughly halfway between throughfeed strings 17. In figure 4 the throughfeed mechanism 16 is only shown at the position of inlet opening 4. The skilled person will appreciate that throughfeed mechanism 16 can be embodied in wholly analogous manner at the position of outlet opening 5. The description below, which describes the shown throughfeed mechanism 16 at a position of the inlet opening, will therefore also serve fully as a basis for the throughfeed mechanism at the position of the outlet opening.

The throughfeed strings 17 are trained along respective guide rollers 18 at the position of the inlet opening so that the direction of movement of throughfeed strings 17 is changed. As shown in figure 4, this can also take place multiple times in order to segment a greater change in direction of movement into smaller angles. Further guide rollers 18 (not shown) can also be provided in order to realize a change in direction of movement in an opposite direction. A loading zone is provided at a distance from inlet opening 4. Face masks can be connected to throughfeed strings 17 at the position of the loading zone. Use is preferably made for this purpose of frames as shown in figure 3 and described above. Alternatively, throughfeed strings 17 themselves are provided with protrusions and the face masks are connected directly to throughfeed strings 17. An unloading zone can be provided at a distance from outlet opening 5.

The skilled person will appreciate on the basis of the above description that the invention can be embodied in different ways and on the basis of different principles. The invention is not limited to the above described embodiments. The above described embodiments and the figures are purely illustrative and serve only to increase understanding of the invention. The invention will not therefore be limited to the embodiments described herein, but is defined in the claims.

## Claims

1. Device for cleaning face masks, the device comprising a housing with an inlet opening and an outlet opening, wherein a throughfeed mechanism is provided for carrying the face masks from the inlet opening to the outlet opening along a throughfeed path, wherein cleaning means are provided in the housing all around the throughfeed path in a manner such that the cleaning has an effect from substantially all perpendicular directions of incidence to the throughfeed path, wherein the device further comprises an inlet screening and outlet screening comprising a supply opening and a discharge opening, wherein the throughfeed mechanism is further provided to carry the face masks from the supply opening to the inlet opening and is provided to carry the face masks from the outlet opening to the discharge opening, wherein the supply opening and the discharge opening lie at a distance from the throughfeed path in order to screen a surrounding area from the cleaning means, wherein the face masks each have on either side a fastening element and wherein the throughfeed mechanism has for each face mask at least two, preferably at least three protrusions at a mutual distance, such that the face mask can be connected via its fastening elements to the protrusions so as to be suspended between the protrusions, wherein the frame is formed as a framework, wherein the protrusions are provided on the framework so that the face mask can be suspended within boundaries of the framework, wherein the throughfeed mechanism comprises two parallel throughfeed strings between which the face masks can be secured, wherein the transport means comprises the throughfeed strings.

2. Device according to claim 1, wherein the throughfeed mechanism comprises a plurality of frames with the protrusions, wherein each frame is provided to hold a predetermined number of face masks, wherein the throughfeed mechanism further comprises a transport means to which the frames can be connected in order to move the frames with the face masks along the throughfeed path.

3. Device according to claim 1 or 2, wherein the frame is magnetically compatible with the transport means in order to connect the frame magnetically to the transport means.

4. Device according to any one of the foregoing claims, wherein the throughfeed mechanism comprises at the position of each of the inlet opening and the outlet opening at least one bend element for changing a throughfeed direction.

5. Device according to any one of the foregoing claims, wherein the cleaning means comprises at least one UV-C lamp.

6. Device according to any one of the foregoing claims, wherein the at least one UV-C lamp is mounted movably in the housing such that the UV-C lamp is movable all around the central axis so as to change an angle of incidence of radiation of the UV-C lamp onto the throughfeed path.

7. Device according to the foregoing claim, wherein the at least one UV-C lamp comprises a whole number N of UV-C lamps, wherein N is greater than or equal to 2 and wherein the UV-C lamps are positioned around the central axis with an angular intermediate distance of 360 degrees divided by N.

8. Device according to the foregoing claim, wherein the N UV-C lamps are mounted on a structure which is bearing-mounted rotatably in the housing, which structure is provided for reciprocal rotation about the central axis between two end positions lying at a mutual angular intermediate distance of at least 360 degrees divided by N.

9. Device according to any one of the foregoing claims, wherein the housing further comprises a sensor for measuring an operating intensity of the cleaning means.

10. Device according to the foregoing claim, wherein the sensor is operatively coupled to the throughfeed mechanism so as to at least partially control a throughfeed speed on the basis of an output of the sensor.

11. Device according to any one of the foregoing claims, wherein a loading zone and an unloading zone are provided adjacently of respectively the supply opening and the discharge opening.

12. Method for cleaning face masks, the method comprising of:
- feeding the face masks through a housing using a throughfeed mechanism from an inlet opening to an outlet opening along a throughfeed path;
- cleaning in the housing via cleaning means which are provided all around the throughfeed path in a manner such that the cleaning has an effect from substantially all perpendicular directions of incidence to the throughfeed path;
- screening off the cleaning means via an inlet screening and outlet screening comprising a supply opening and a discharge opening, wherein the throughfeed further comprises of carrying the face masks from the supply opening to the inlet opening and from the outlet opening to the discharge opening, wherein the supply opening and the discharge opening lie at a distance from a central axis of the throughfeed path.

13. Method according to the foregoing claim, further comprising of measuring an operating intensity of the cleaning means during throughfeed.

14. Method according to the foregoing claims 12 or 13, wherein the cleaning means comprises a whole number N of UV-C lamps, wherein N is greater than or equal to 2 and wherein the UV-C lamps are positioned around the central axis with an angular intermediate distance of 360 degrees divided by N, and wherein the step of cleaning comprises of reciprocally rotating the N UV-C lamps about the central axis through an angular distance of at least 360 degrees divided by N.
